# EUROPEAN PATENT SPECIFICATION

(11) **EP 3 544 554 B1**
(45) Date of publication and mention of the grant of the patent: **17.04.2024**
(21) Application number: 17812173.7
(22) Date of filing: 22.11.2017
(51) Int. Cl.: A61F 5/44

(54) **DRAINAGE SYSTEM FOR OSTOMY POUCH**
DRAINAGESYSTEM FÜR OSTOMIEBEUTEL
SYSTÈME DE DRAINAGE POUR POCHE DE STOMIE

(30) Priority: 22.11.2016 US 201662425429 P
(43) Date of publication of application: 02.10.2019
(73) Proprietor: Hollister Incorporated, Libertyville, IL 60048 (US)
(72) Inventor: SCALISE, Anthony, Libertyville, IL 60048 (US); VISCONTI, Peter, L., Libertyville, IL 60048 (US); AUGUSTYN, Christina, Libertyville, IL 60048 (US); TORSTENSEN, Jan, Libertyville, IL 60048 (US); GRUM-SCHWENSEN, Christen, Libertyville, IL 60048 (US); NEILSEN, Kenneth, Libertyville, IL 60048 (US); CISKO, George, J., Jr., Libertyville, IL 60048 (US); TETZLAFF, Patrick, C., Libertyville, IL 60048 (US); LEADINGHAM, Brian, T., Libertyville, IL 60048 (US)
(74) Representative: FRKelly
(86) International application number: PCT/US2017/062964
(87) International publication number: WO 2018/098242

(56) References cited:
- WO-A1-2009/021520
- WO-A1-2013/022575
- US-A- 4 106 507
- US-A- 4 238 059
- US-A1- 2012 130 329
- US-A1- 2014 371 699

## Description

### BACKGROUND

The following description relates generally to ostomy appliances, and in particular, a drainage system for an ostomy pouch to provide additional capacity.

Ostomy pouches for collecting bodily waste are used by persons who have had surgery such as a colostomy, ileostomy, or urostomy. Ostomy pouches typically include flat, opposing side walls defining an internal collection cavity. One of the side walls is provided with an opening to receive a stoma, and means to secure the pouch to the user, such as an adhesive barrier, so that bodily waste discharged through the stoma is received within the cavity without leakage from the stoma/barrier/pouch environment.

The ostomy pouch may be a drainable pouch having a discharge opening at a lower end. The discharge opening may be closed during collection of body waste material, but may be opened to drain the waste material from the pouch. Such drainable ostomy pouches are disclosed, for example, in Nolan, U.S. Pat. No. 3,523,534, and Jensen et al., U.S. Pat. No. 4,411,659.

The discharge opening of a drainable ostomy pouch is typically defined at the end of a narrowed neck portion, which is provided with closure means for maintaining the discharge opening in a sealed condition until waste material is to be drained from the pouch. The closure means may take the form of a clamp, as in the aforementioned Nolan patent, or a device such as conventional wire ties or wraps for securing the neck portion in an upwardly-rolled condition.

In daily use, bodily waste may accumulate in the ostomy pouch. The capacity of the ostomy pouch is limited, and the pouch will need to be drained as the bodily waste accumulates to a level approaching a capacity of the pouch. Bodily waste may be drained from the ostomy pouch by opening the discharge opening. However, for night use, such occasional draining may not be convenient for the patient as it may interrupt sleep.

Bedside, or night, drainage systems have been developed to allow for an increased volume of bodily waste to be collected. Such drainage systems are disclosed, for example, in Fenton, U.S. Pat. No. 3,825,005, Temple, U.S. Pat. No. 5,356,400, and Ryder et al., U.S. Pat. App. Pub. No. 2010/0152686. The bedside drainage systems typically include a hose or tube to be coupled to the discharge opening at one end and a night reservoir at the other end. For example, as described in Fenton, a drainable pouch may be provided with exterior ribs on the discharge port for connecting to another collecting device via a sleeve having mating ribs. In another system, such as that described in Temple, a tube is connected to a collection bag with a connector in the form of two adhesively coated, resilient, fluid impervious pieces of sheet material.

However, in the configurations above, the connections between the hose or tube and the collection bag may be difficult to manufacture and/or difficult for a user to operate. Additionally, in the aforementioned systems, bodily waste may become lodged in the hose or tube, which requires further attention from the wearer or caretaker to prevent over filling of the collection bag. Further, in the systems above, the bedside drainage bag may need to be secured to, or supported on, a surrounding structure with an external fastener. However, it may be difficult or inconvenient to remove and attach the fastener from the bedside drainage bag whenever the bedside drainage bag is to be emptied or replaced.

Accordingly, it is desirable to provide a drainable ostomy pouch system having convenient couplings between a bedside drainage bag, hose and ostomy pouch, improved flow capabilities through the hose, and a hanger or fastener for securing or supporting the bedside drainage bag on an adjacent structure, such as a bed frame.

WO2013022575A1 may be considered to represent the closest prior art, it discloses an ostomy appliance including a dual-chamber pouch, which can continuously drain liquids from body waste, while collecting solids in the pouch. The ostomy appliance can also include a flat output tap including a lumen, the size of which can be increased by applying pressure.

US4238059A discloses a stoma drainage appliance having a fluid collecting pouch constructed of sheets of flexible material with two internal sheets sealed together at spaced intervals to provide a valve across the center of the pouch precluding upward flow of fluid between the two internal sheets. There is at least one unsealed space between the seals located so as to provide a direct, straight path from an inlet opening of the upper section of the pouch to the lower section of the pouch. The stoma drainage appliance further includes an outlet spout connected at the lower section of the pouch. The spout has a tapered opening with an inlet neck section of oval cross-section and an outlet end section of circular cross-section. The spout is made of flexible material for clamping along the neck section to close the spout and includes a cap for attachment to the end section thereof. A tubular coupling member is provided for connecting to the spout and it has an opening of tapered construction to accommodate external conduits of varying size.

US2012130329A1 discloses a valve that is sealable to an associated container having an interior fluid storage region is adapted to permit and stop flow of fluid from the fluid storage region. The valve includes a body sealable to the container and a stem mounted to the body having a portion in flow communication with the storage region. The stem is movable relative to the body between an open position to permit flow from the storage region through the valve, and a closed position to stop flow through the valve. The stem has a grasping region spaced from the body that is adapted rotate the stem to move the valve between the open and closed positions. The grasping region has first and second sides. One of the sides has a tactile indicator to provide indication of the position of the valve, the other side is devoid of the tactile indicator.

### SUMMARY

The present disclosure provides a drainable ostomy system as detailed in claim 1. Advantageous features are provided in dependent claims.

According to one embodiment, a drainable ostomy system includes an ostomy pouch having an internal collection area and an outlet for draining contents from the collection area, and an interface assembly. The interface assembly includes an outlet body operably connected to the outlet, a cap configured for removable coupling with the outlet body and configured to close a distal end of the outlet body, and a bedside drainage adapter configured for removable coupling with the outlet body, wherein the cap and bedside drainage adapter are interchangeably coupled to outlet body.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a diagram showing a drainable ostomy system according to an embodiment described in herein;
FIGS. 2-5 show examples of the drainable ostomy system of FIG. 1 in different stages of use;
FIGS. 6-8 show examples of a bedside drainage bag according to an embodiment described herein;
FIGS. 9-14 show examples of a drainable pouch outlet body with a cap and a bedside drainage adapter, according to an embodiment described herein;
FIG. 15 shows an example of another drainable pouch outlet body and cap, according to an embodiment described herein;
FIGS. 16 and 17 show examples of another drainable pouch outlet body and cap, according to an embodiment described herein;
FIGS. 18-20 show examples of another drainable pouch outlet body and cap, according to an embodiment described herein;
FIGS. 21-25 show examples of another drainable pouch outlet body, cap and bedside drainage adapter, according to an embodiment described herein;
FIGS. 26-28 show examples of another drainable pouch outlet body, cap and bedside drainage adapter, according to an embodiment described herein;
FIGS. 29-43 show examples of another drainable pouch outlet body, cap and bedside drainage adapter, according to an embodiment described herein;
FIG. 44 shows an example of another drainable pouch outlet body and cap, according to an embodiment described herein;
FIGS. 45-47 show examples of another drainable pouch outlet body and cap, according to an embodiment described herein;
FIGS. 48-50 show examples of another drainable pouch outlet body and cap, according to an embodiment described herein;
FIG. 51 and 52 show examples of the drainable pouch outlet body of FIG. 48 together with a bedside drainage adapter, according to an embodiment described herein;
FIGS. 53-55 show examples of valves configured for use with an outlet body of an ostomy pouch, according to embodiments described herein;
FIGS. 56 and 57 show examples of another a drainable outlet body and cap according to an embodiment described herein;
FIGS. 58-60 show examples of another drainable outlet body, cap and bedside drainage adapter according to an embodiment described herein;
FIGS. 61 and 62 show examples of another drainable outlet body according to an embodiment described herein;
FIG. 63 is a cross-sectional view showing another example of a drainable outlet body according to an embodiment described herein; and
FIG. 64 is a cross-sectional view showing another example of a drainable outlet body and bedside drainage adapter according to an embodiment described herein.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated. The invention is solely defined by the scope of the appended claims.

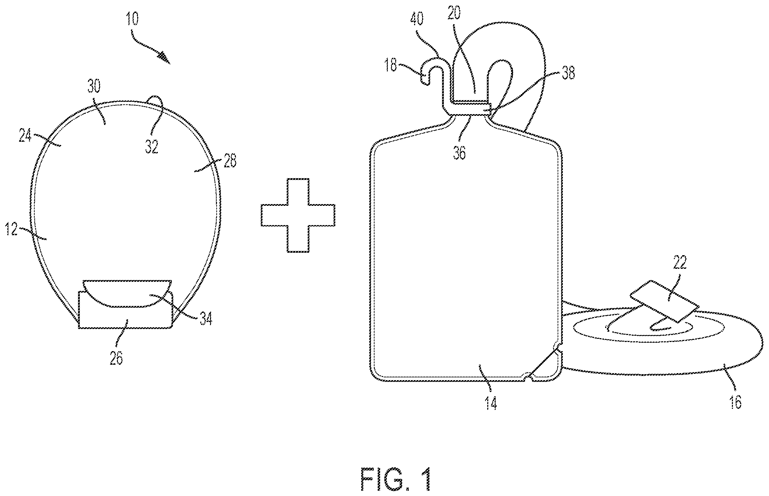

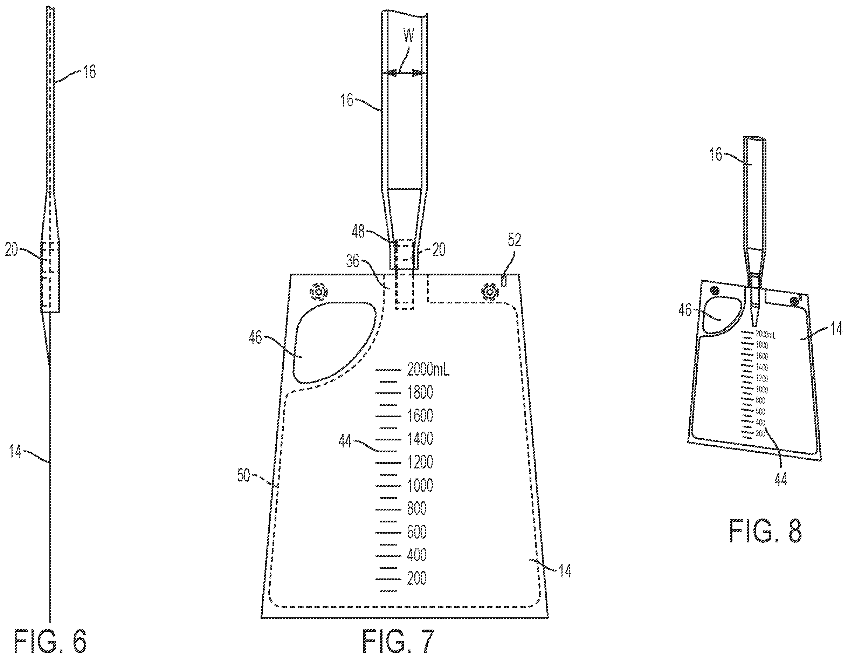

Referring to FIG. 1, a drainable ostomy system 10, according to the embodiments described herein, generally includes an ostomy pouch 12, a bedside drainage bag 14 and a tube 16 configured to connect the ostomy pouch 12 to the bedside drainage bag 14. In one embodiment, the bedside drainage bag 14 may have a hanger 18 configured to engage an adjacent structure and support the bedside drainage bag 14 on the structure. In addition, the drainable ostomy system 10 may include a bag connector 20 (see FIGS. 6 and 7) to connect the tube 16 to the bedside drainage bag 14 and an interface assembly 22 to selectively connect the tube 16 to the ostomy pouch 12 or, alternatively, substantially close or seal the outlet 26.

Referring still to FIG. 1, the ostomy pouch 12 may include a collection pouch 24 and an outlet 26 for draining contents from the collection pouch 24. The collection pouch 24 defines an internal collection area 28. The collection pouch 24 may generally be formed by and between a first side wall 30 and a second side wall 32 joined to one another at a periphery, or connected integrally with one another as a single unit. The collection area 28 is defined between the first and second side walls 30, 32. The ostomy pouch 12 also includes an inlet (not shown) formed in one of the side walls 30, 32 configured to receive a stoma so that a discharge from the stoma may be received in the collection area 28. The inlet may be positioned at an upper portion of the collection pouch 24. The outlet 26 may be disposed generally at a lower end of the collection pouch 24 to allow for drainage of contents from the collection area 28.

In one embodiment, the outlet 26 may be formed as a drainage tail 26 extending from a lower end of the collection pouch 24 and having a narrowed width relative to the collection pouch 24. The drainage tail 26 is movable between an open condition and a closed condition. In the open condition, the drainage tail 26 is unfolded or unrolled to provide a substantially unobstructed internal passageway for the contents of the collection area 28 to egress from the collection pouch 24 through the outlet 26. The open condition may be used to drain or clean the ostomy pouch 12, or when the ostomy pouch 12 is used in conjunction with the bedside drainage system 10. The bedside drainage system 10 may be desirable, for example, for night time use, extended periods of time of bed rest or other times when the wearer is generally stationary.

The drainage tail 26 may be moved to the closed condition by upwardly rolling or folding the tail 26. This upward rolling or folding closes or seals the internal passageway to substantially limit or prevent egress of contents from the collection area 28 through the drainage tail 26. Maintaining the ostomy pouch 12 in the closed condition may be desirable, for example, during normal daily use or while the wearer is travelling or otherwise mobile.

In one embodiment, the drainage tail 26 may be held in the closed condition by one or more fasteners 34. That is, the one or more fasteners 34 may substantially secure the drainage tail 26 from inadvertent movement from the closed condition to the open condition. In one embodiment, the one or more fasteners 34 may include, for example, a hook-and-loop fastener such as VELCRO ^{®}. Alternative, or additional, fasteners may be used as well, such as wires, adhesives, interlocking fasteners and the like.

The bedside drainage bag 14 is connected to the ostomy pouch 12 by the tube 16 such that contents from the ostomy pouch 12 may be drained into and stored in the bedside drainage bag 14 through the tube 16. Thus, the bedside drainage bag 14 serves to provide increased storage capacity to store the discharged bodily waste. In one embodiment, the tube 16 has an internal width 'W' or diameter sufficient to substantially deter blocking and stoppages of contents within the tube 16, to allow for substantially unrestricted flow of contents from the ostomy pouch 12 to the bedside drainage bag 14. In one embodiment, the width 'W' may be approximately 40 mm, but is not limited thereto.

The bedside drainage bag 14 includes an inlet 36, for example, at an upper end, to receive contents from the ostomy pouch 12 via the tube 16. The bedside drainage bag 14 also includes the hanger 18. The hanger 18 may be formed integrally as one piece with the bedside drainage bag 14, for example, in a molding process, including 2-shot injection molding, or, alternatively, may be formed separately from the bedside drainage bag 14 and secured thereto. The hanger 18 may be secured to the bedside drainage bag 14, for example, by heat sealing, adhesive, hook-and-loop fastener or other suitable fastening technique. In one embodiment, the inlet 36 may be formed at a narrowed neck portion of the bedside drainage bag 14 and the hanger 18 may be secured at the neck. Further, in one embodiment, the hanger 18 may include a ring 38 extending around the neck. The hanger 18 may also include a hook 40 configured to engage an adjacent object such that the bedside drainage bag 14 may be connected or attached to the object. In one embodiment, the hanger 18 may attach the bedside drainage bag 14 to a bedframe in such a way that the bedside drainage bag 18 hangs from the bedframe.

In one embodiment, the tube 16 is connected to the bedside drainage bag 14 with the bag connector 20. The bag connector 20 may be formed integrally with one of the tube 16 and the bedside drainage bag 14, or have portions integrally connected to both. Alternatively, the bag connector 20 may be secured to one of or both of the tube 16 and the bedside drainage bag 14. Preferably, the bag connector 20 forms a sealed connection between the bedside drainage bag 14 the tube 16.

According to one embodiment, the tube 16 may be connected to the ostomy pouch 12 with the interface assembly 22. The interface assembly 22 may be disposed at the outlet 26 of the ostomy pouch 12 and/or one end of the tube 16. As described further below, the interface assembly 22 may include one or more components configured to couple to each other, or with one or more of the outlet 26 and the tube 16 to form a sealed connection between the outlet 26 and the tube 16, or to close the outlet 26.

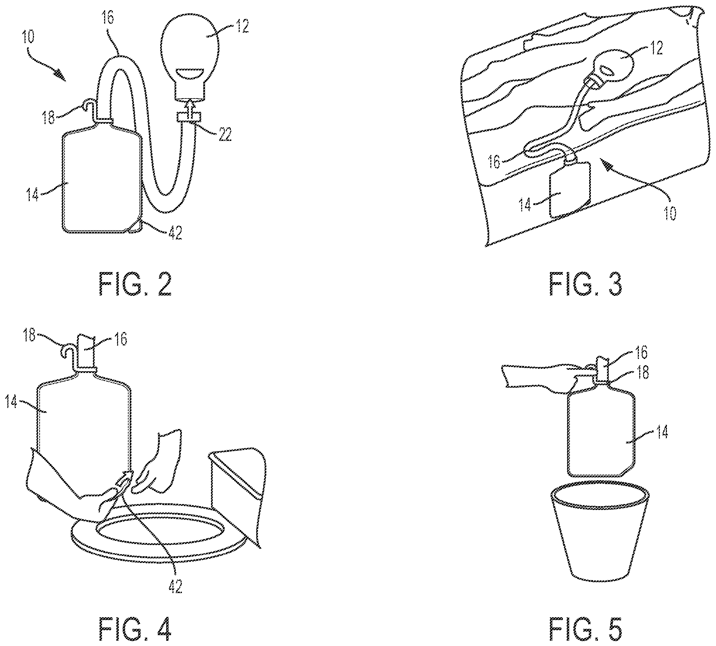

FIGS. 2-5 show examples of the drainable ostomy system 10 in use, according to an embodiment described herein. With reference to FIG. 2, the tube 16 may be coupled, at one end, to the bedside drainage bag 14 and coupled, at another end, to the ostomy pouch 12. Referring to FIG. 3, the bedside drainage bag 14 may be hung, or otherwise connected to or supported on, an adjacent structure, such as a bed or bedframe, while the ostomy pouch 12 is secured to the peristomal area of, and supported on, the wearer for regular use. Referring to FIG. 4, the bedside drainage bag 14 may be emptied or cleaned. For example, the bedside drainage bag 14 may have a resealable or recloseable bag outlet 42 through which contents may be emptied. The bag outlet 42 may be formed as, or additionally include a tear-away feature, such as a perforation or tear line to allow tearing away of a portion of the bedside drainage bag 14 to empty the bag 14. Referring to FIG. 5, the bedside drainage bag 14 may be disposed of. The hanger 18 may also serve as a handling mechanism.

FIGS. 6-8 show different views of the bedside drainage bag 14 together with the tube 16, according to an embodiment described herein. In one embodiment, the bedside drainage bag 14 may be substantially transparent or translucent, or include a transparent or translucent section allowing for visual inspection of an interior of the bedside drainage bag 14 through a sidewall of the bag 14. In addition, the bedside drainage bag 14 may include volume markers 44 so that a volume of the contents (e.g., bodily fluid) received in the bedside drainage bag 14 may be readily determined. The volume markers 44 may be formed on the transparent or translucent section. Further, the bedside drainage bag 14 may be formed having a loop or opening 46 to allow for handling of the bag 14. In one embodiment, the bedside drainage bag 14 may include two opposing sidewalls secured together around or near their respective perimeters. The opposing sidewalls may be secured together, for example, by heat sealing to form a peripheral seam or joint 50. Alternatively, a one-piece construction may be used. A pre-cut section 52 may be formed in bedside drainage bag 14 to allow for tearing away a section to empty the contents from the bag 14.

Referring to FIGS. 7 and 8, in one embodiment, the bag connector 20 may be inserted in the inlet 36 of the bedside drainage bag 14 and an outlet end of the tube 16. In one embodiment, the bag connector 20 is heat sealed in the inlet 36 of the bedside drainage bag 14 and has a section extending outwardly from the bedside drainage bag 14. The tube 16 may be secured on the outwardly extending section of the bag connector 20. Preferably, the tube 16 tightly fits around the bag connector 20 to form a sealed connection therewith. The bag connector 20 may also include a collar 48 having an increased diameter to assist in sealing against an inner wall of the tube 16. Alternatively, the outlet end of the tube 16 may be received within a portion of the bag connector 20. In still another example, the tube 16 and inlet 36 of the bedside drainage bag 14 may be directly coupled to one another, or formed as single, integral part.

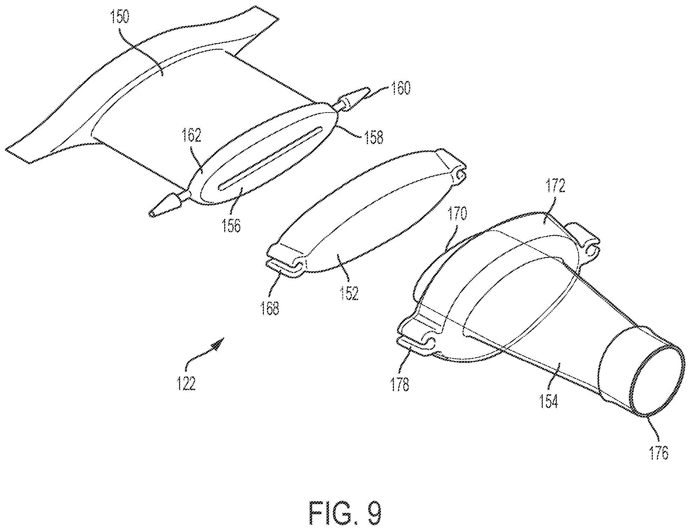

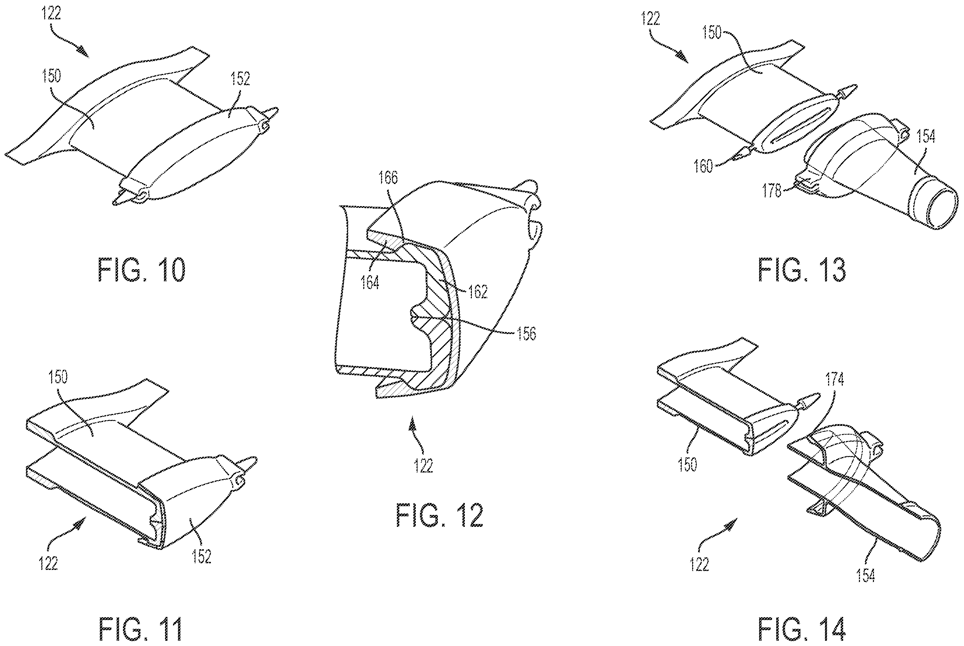

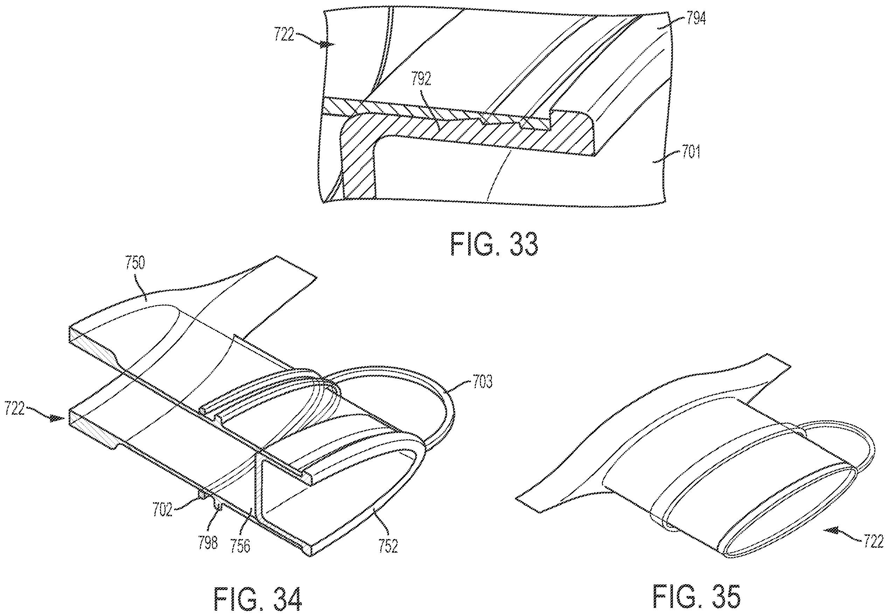

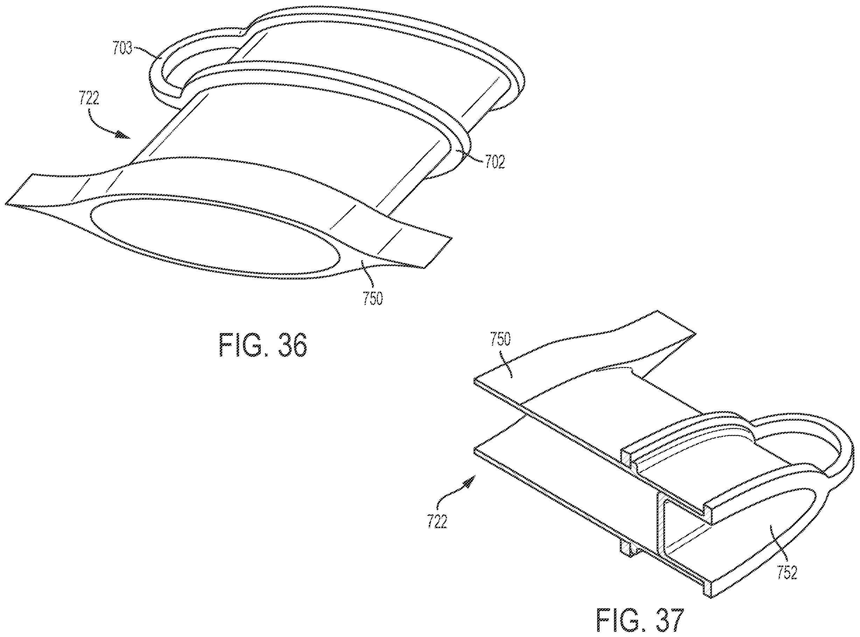

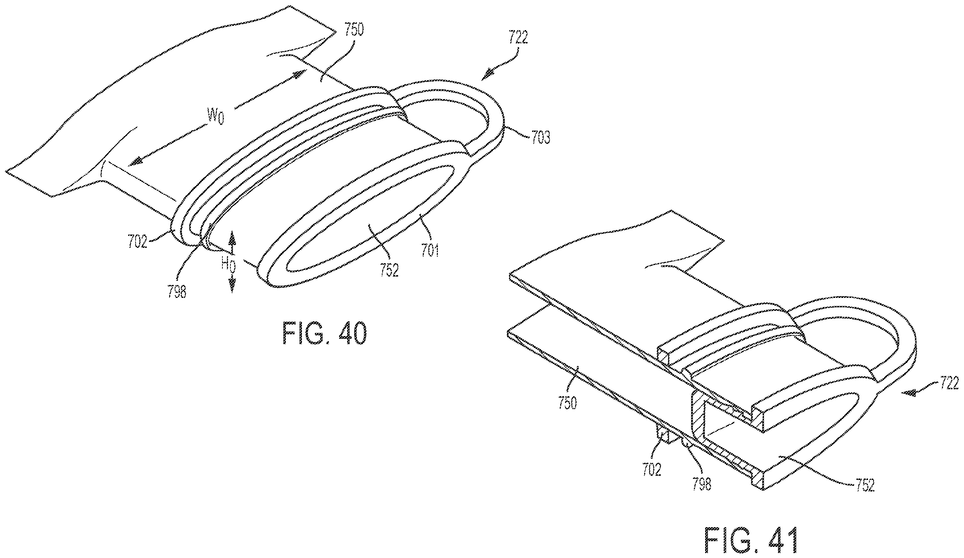

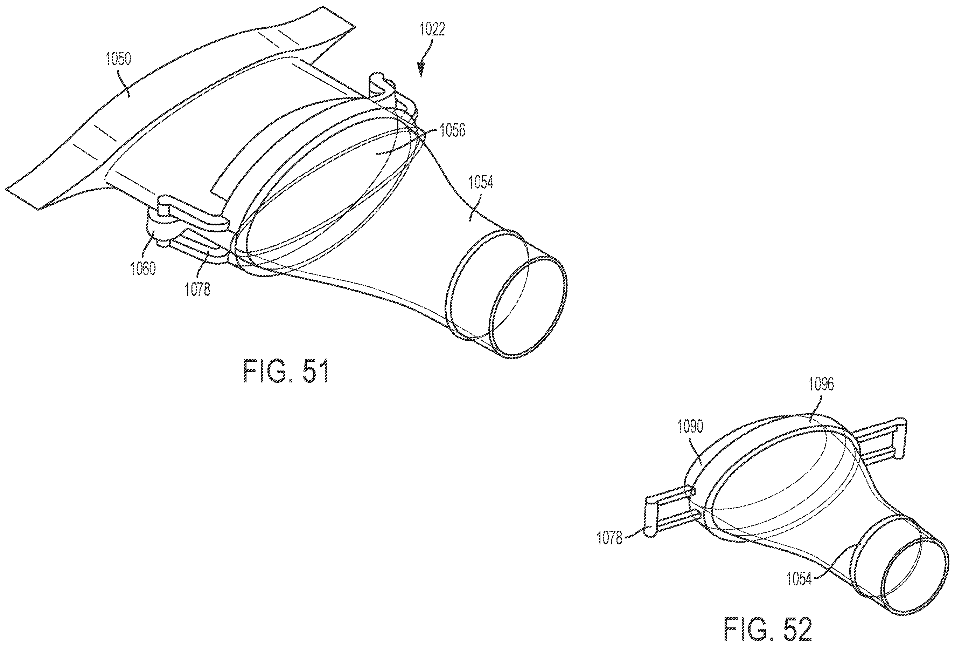

FIGS. 9-64 show various embodiments of the interface assembly 22. In general, the interface assembly 22 includes an outlet body, a cap and a bedside drainage adapter and is configured for positioning between the outlet 26 of the ostomy pouch 12 and the tube 16. The outlet body is connected to the outlet 26. The cap and the bedside drainage adapter are removably and interchangeably connected to the outlet body, such that in one embodiment, either the cap or the bedside drainage adapter is coupled to the outlet body. The bedside drainage adapter is connected to the tube 26. In one embodiment, the cap and the bedside drainage adapter may be removable from the outlet body and interchangeable with one another depending on a condition, i.e., open or closed, the outlet 26 is in, and a desired functionality, e.g., closed for collecting bodily waste in the ostomy pouch 12 or open to drain the ostomy pouch 12 into the bedside drainage bag 14. It is understood, that in the following embodiments, the outlet body may be implemented as an independent component. For example, the outlet body may be implemented as a component of the outlet 26, and may be adapted for use with the cap or the bedside drainage adapter. The outlet body may be used in conjunction with the outlet 26, and independently of the bedside drainage adapter, to control emptying or draining of the contents from the ostomy pouch 12.

FIGS. 9-14 show an interface assembly 122 according to an embodiment described herein. The interface assembly 122 includes an outlet body 150, a cap 152 and a bedside drainage adapter 154 interchangeably positioned with the cap 152 for coupling to the outlet body 150, according to an embodiment described herein. The outlet body 150 may be disposed at least partially within the outlet 26 and have a slit or opening 156 formed at a distal, or discharge, end 158. In one embodiment, the outlet body 150 may be heat sealed to, or within, the outlet 26. Alternatively, the outlet body 150 may be molded, including 2-shot injection molded, adhered, or otherwise suitably connected to the outlet 26. The slit 156 at the distal end 158 is urged to a closed position to substantially prevent or limit egress of collection pouch contents through the outlet body 150. To this end, the slit 156 may be urged to a closed condition by an intrinsic biasing force in the material from which the outlet body 150 is formed and/or a geometry of the slit 156 and the outlet body 150. Alternatively, or additionally, the outlet body 150 may include a biasing element (not shown) to urge the slit 156 toward its closed condition. The outlet body 150 may also include a first latch 160 and a first shoulder 162. The distal end 158, including the slit 156, may generally be formed as a squeeze valve, such that when external forces are applied in a predetermined direction or directions, the slit 156 may open to allow egress of the contents from the collection pouch 24. Release of the external forces allows the distal end 158, including the slit 158, to return to its closed condition, where egress of the contents is substantially prevented.

The cap 152 may be removably disposed over the discharge end 158 of the outlet body 150 and include a second shoulder 164 configured to releasably engage the first shoulder 162. The engagement between the first shoulder 162 and second shoulder 164 is configured to form a perimeter seal 166 on or near the distal end 158. One of, or both, the cap 152 and the outlet body 150 may be formed of a resilient material such that the first and/or second shoulder 162, 164 deflects as one moves over the other, and are urged toward one another to move to the engaged position after clearing one another to form the perimeter seal 166. The cap 152 may also include a second latch 168 configured to releasably engage the first latch 160 to further secure the cap 152 to the outlet body 150 against unintentional removal. In one embodiment, one of the first latch 160 and the second latch 168 may be a resilient arm movable into an interlocking, snap or friction fit, or the like, with a groove or receptacle of the other of the first latch 160 and second latch 168. With the cap 152 secured on the outlet body 150, the ostomy pouch 12 may be in a substantially closed condition to collect and store bodily waste therein. This condition generally corresponds with a rolled-up or closed condition of the outlet 26. However, the cap 152, disposed on the outlet body 150, may also substantially prevent egress of the contents from the ostomy pouch 12 when the outlet 26 is unrolled or moved to an open condition.

The cap 152 may be removed from the outlet body 150 and replaced with the bedside drainage adapter 154. In one embodiment, the bedside drainage adapter 154 includes an insertion end 170 configured to be received within the slit 156. A force, for example, opposing inwardly directed forces, may be applied to the outlet body 150 to move the slit 156 to an open position such that the insertion end 170 may be received in the slit 156. The bedside drainage adapter 154 may also include a collar 172 configured for fitting around an exterior of the outlet body 150. The collar 172 may include a third shoulder 174 for engaging the first shoulder 162 in a manner similar to the second shoulder 164 of the cap 152. Accordingly, the perimeter seal 166 may alternatively be formed by engagement of the first and third shoulders 162, 174. An outlet end 176 of the bedside drainage adapter 154, opposite to the insertion end 170, may be coupled to, or formed integrally with, the tube 16. The bedside drainage adapter 154 includes an internal passageway to fluidically connect the collection area 28 of the ostomy pouch 12 to the tube 16 so that contents may be drained from the collection area 28. The bedside drainage adapter 154 may also include a third latch 178 configured to releasably engage the first latch 160 of the outlet body 150.

In use, according to the embodiments described herein, the cap 152 may be secured on the outlet body 150 when it is desired for the ostomy pouch 12 and the outlet 26 to be in a sealed, closed condition, for example, during normal daily use. The outlet 26 may be moved to the open condition for connection to the bedside drainage bag 14. The cap 152 may be removed and replaced with the bedside drainage adapter 154 which is connected to the tube 16, and in turn, to the bedside drainage bag 14. Accordingly, contents from the ostomy pouch 12 may be drained into the bedside drainage bag 14.

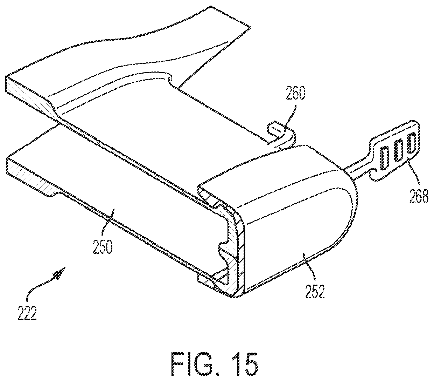

FIG. 15 is a perspective view showing another embodiment of an interface assembly 222 comprising an outlet body 250 and a cap 252. In this embodiment, the cap 252 may include a second latch 268. The second latch 268 may be formed as a resilient arm configured to engage a first latch 260. The first latch 260 may be formed as a hook or clasp on the outlet body 250.

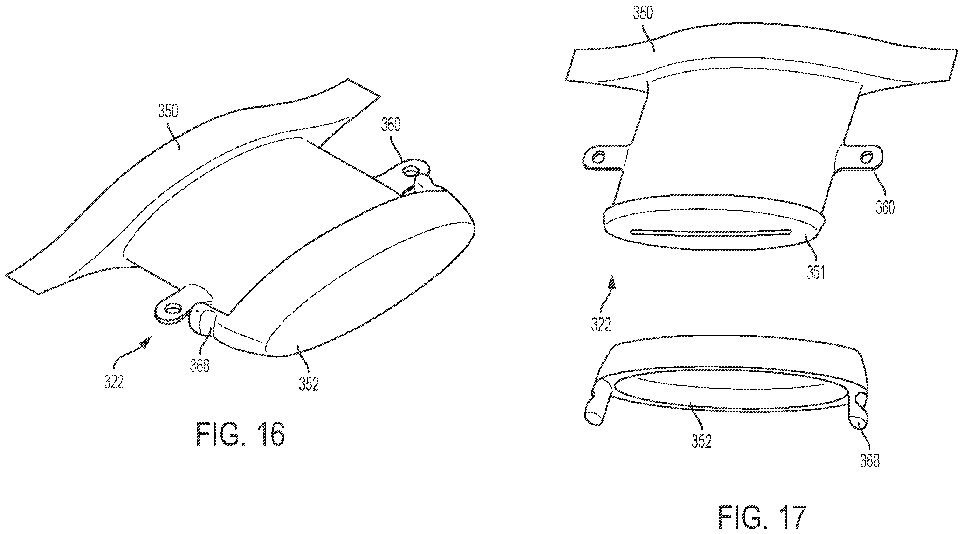

FIGS. 16 and 17 show an interface assembly 322 having an outlet body 350 and a cap 352 according to another embodiment described herein. Referring to FIGS. 16 and 17, the outlet body 350 may include a first latch 360. The first latch 360 may be formed as one or more flexible lugs, each lug having an opening therein. The cap 352 may include a second latch 368. The second latch 368 may include an arm having a detent or groove formed therein. The cap 352 may be fitted over the distal end 351 of the outlet body 350, and preferably, forms a sealed or substantially sealed connection around an outer periphery of the outlet body 350. The cap 352 may be removed from the outlet body 350 and replaced with a bedside drainage adapter in the manner described above with respect to FIGS. 9-14.

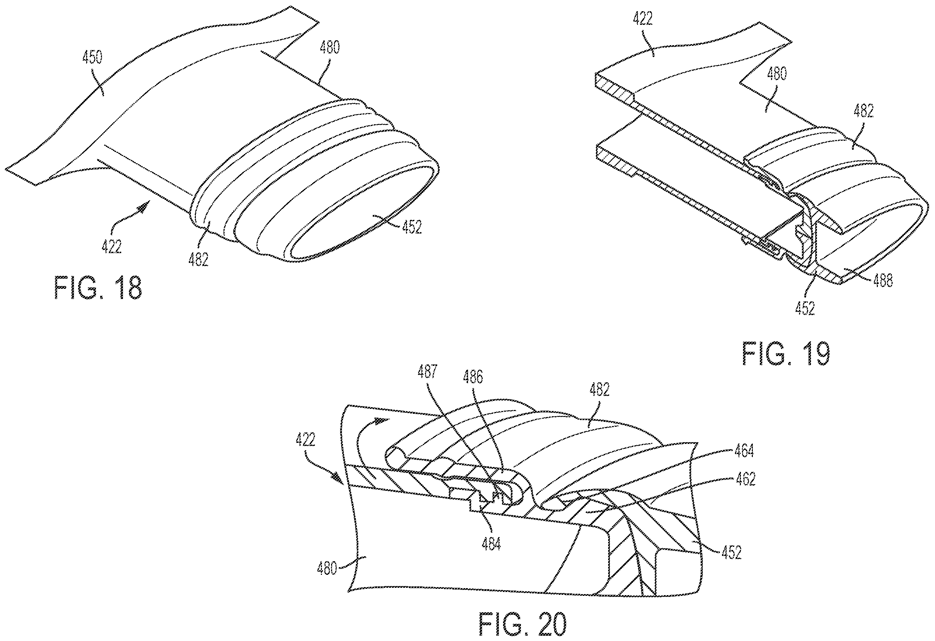

FIGS. 18-20 show an interface assembly 422 having an outlet body 450 and a cap 452 according to another embodiment described herein. Referring to FIGS. 18-20, the outlet body 450 may be formed as a two piece body having a base 480 and an end part 482. In one embodiment, the end part 482 may include an inner leg 484 configured to engage an inner wall of the base 480 and an outer leg 486 configured to engage an outer wall of the base 480. In one embodiment, the inner wall of the base 480 and the inner leg 484 of the end part 482 are each formed with a substantially mating, interlocking structure 487 for attaching the base 480 to the end part 482. The end part 482 may also include a slit 456 that may be selectively opened and closed, thereby forming a squeeze valve. The cap 452 may be fitted over the end part 482 to seal and close the outlet body 450. In one embodiment, the end part 482 may include a first shoulder 462 and the cap 452 may include a second shoulder 464 configured for mutual engagement substantially as described above. Accordingly, a perimeter seal 466 may be formed at an engagement of the first and second shoulders 462, 464. In one embodiment, an additional sealing material, such as a releasable adhesive, may be disposed at or near one of or both shoulders 462, 464. Further, the cap 452 may include an outwardly projecting (relative to the outlet body 450) collar 488 defining a recess at one end of the cap 452. The cap 452 may be removed from the end part 482 and a bedside drainage adapter according to the embodiments herein may be coupled to the end part 482.

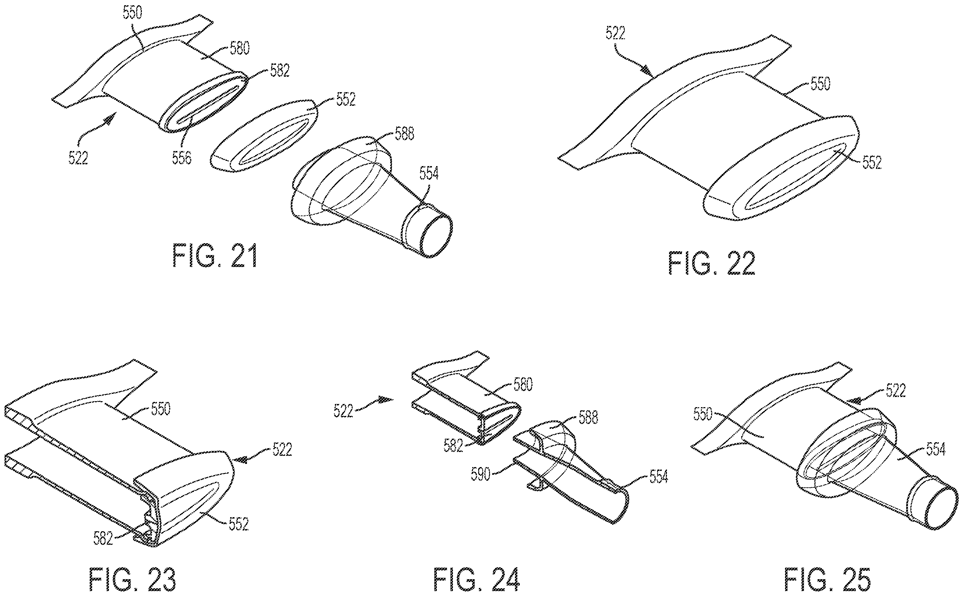

FIGS. 21-25 show an interface assembly 522 having an outlet body 550, a cap 552 and a bedside drainage adapter 554 according to another embodiment described herein. Referring to FIGS. 21-25, the outlet body 550 may be a two piece body having a base 580 and an end part 582, the end part 582 having the slit 556 formed therein to form a squeeze valve as described in the embodiments above. In one embodiment, the end part 582 may be positioned substantially within the base 580 and be substantially flush with an end of the base 580. The cap 552 may fit over the base 580 and end part 582 such that an interior surface of the cap 552 engages an exterior surface of the outlet body 550 to substantially seal the outlet body 550 against unintentional drainage of contents from the ostomy pouch 12. To drain the ostomy pouch 12, the cap 552 may be replaced with the bedside drainage adapter 554. In one embodiment, the slit 556 may be elastically deflected to an open position, for example, by applying opposing inwardly directed forces to the outlet body 550. In the open position, the slit 556 can receive an inner extension 590 of the bedside drainage adapter 554, while an inwardly projecting (relative to the outlet 550) collar 588 of the bedside drainage adapter 554 engages an exterior surface of the outlet body 550. One of, or both of the inner extension 590 and the collar 588 may be substantially annular, for example, in a circular, oval or elliptical shape. The tube 16 may be fitted to an opposite end of the bedside drainage adapter 554.

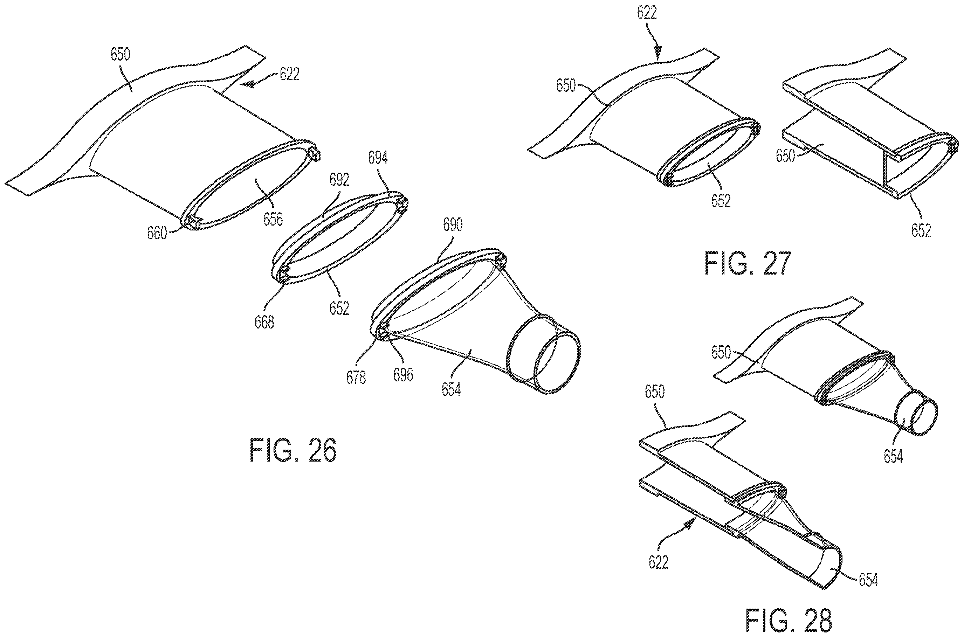

FIGS. 26-28 show an interface assembly 622 comprising an outlet body 650, a cap 652 and a bedside drainage adapter 654 according to another embodiment described herein. Referring to FIGS. 26-28, the outlet body 650 includes an opening 656 at a discharge end and a first latch 660. The cap 652 may be fitted within the opening 656. In one embodiment, the cap 652 includes an inner extension 692 received within the opening 656 and a lip 694 to abut again an axial end face of the outlet body 650. A second latch 668 on the cap 652 may engage the first latch 660 to secure the cap 652 within the opening 656. Preferably, the cap 652 substantially seals the opening 656 against unintentional egress of contents from the ostomy pouch 12. In an open condition, the cap 652 may be replaced with the bedside drainage adapter 654. Similar to the cap 652, the bedside drainage adapter 654 may also include an inner extension 690 configured for positioning within the opening 656 and a lip 696 configured to abut an axial end face of the outlet body 650. The bedside drainage adapter 654 may also include a third latch 678 configured to engage the first latch 660. Preferably, the bedside drainage adapter 654 forms a sealed connection to the outlet body 650.

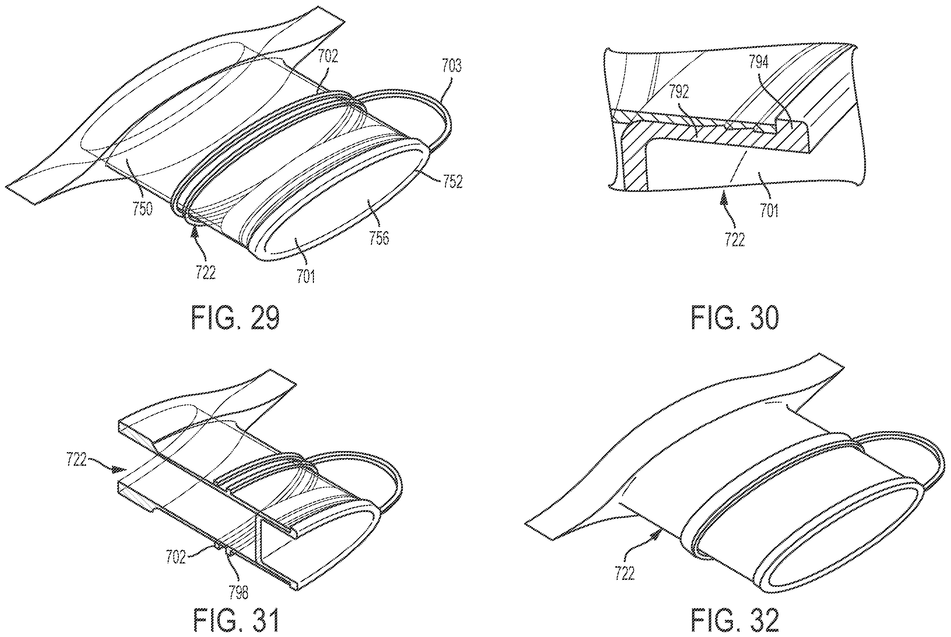

FIGS. 29-43 show an interface assembly 722 comprising an outlet body 750, a cap 752 and a bedside drainage adapter 754 according to another embodiment described herein. Referring to FIGS. 29-43, the outlet body 750 may be formed with an annular ridge 798 on its exterior surface at a position spaced from an end of the outlet body 750 having a discharge opening 756. The cap 752 may include a closure 701 configured for removable positioning in the opening 756 of the outlet body 750. Preferably, the closure 701 seals or substantially seals the outlet body 750 to limit or prevent egress of contents from the ostomy pouch 12 through the outlet body 750. In one embodiment, the closure member 701 may include an inner extension 792 configured for positioning in the opening 756 and lip 794, formed integrally with the inner extension, configured to abut an axial end face of the outlet body 750. The inner extension 792 may include one or more ribs for engaging an inner wall of the outlet body 750. The ribs may assist in providing a sealing engagement between the closure 701 and the outlet body 750.

The cap 752 may further include a mounting loop 702 configured to extend around the outlet body 750. In one embodiment, the mounting loop 702 engages the annular ridge 798 to properly position the cap 752 on the outlet body 750. The mounting loop 702 may be connected to the closure member 701 by a strap 703. In one embodiment, the mounting loop 702 may be made from an elastic material. However, other materials are also envisioned so long as the mounting loop 702 is sized to correspond to an outer circumference or perimeter of the outlet body 750. In one embodiment, the outlet body 750 may have a width 'Wₒ' of approximately 37 mm, but is not limited thereto. In one embodiment, the outlet body 750 may have a height 'Hₒ' of approximately 9 mm, but is not limited thereto.

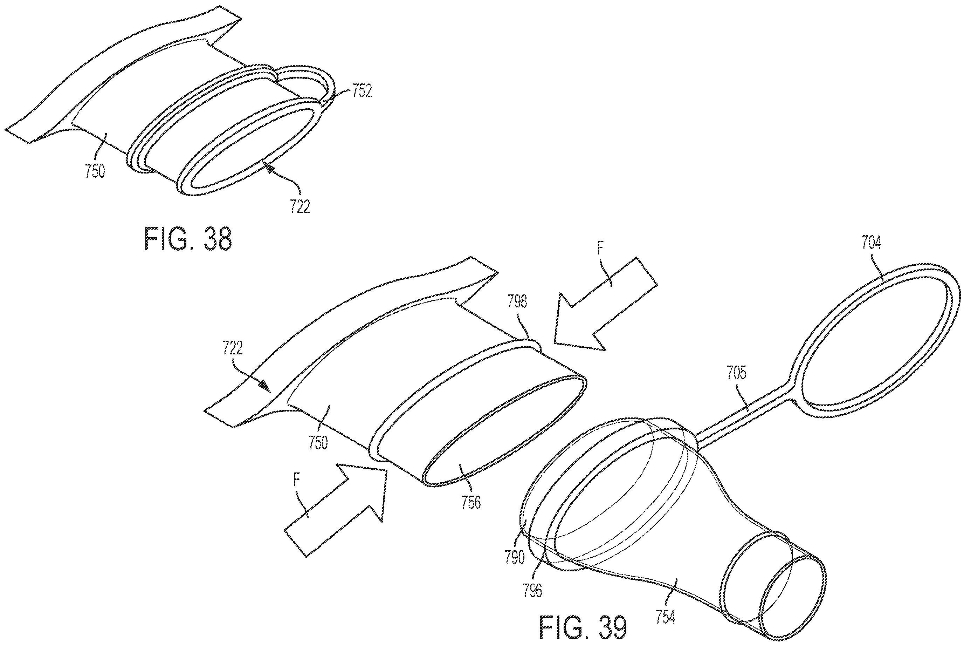

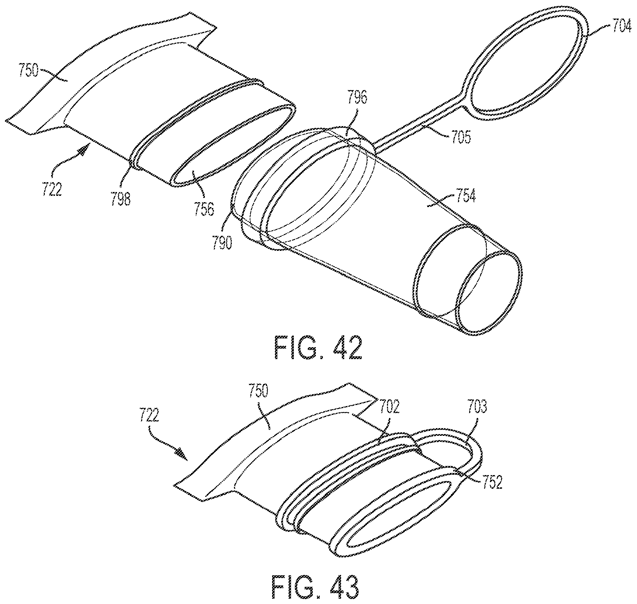

Referring to FIGS. 39 and 42, the cap 752 may be removed and replaced with the bedside drainage adapter 754 to allow for drainage of the ostomy pouch 12 through the outlet body 750. In one embodiment, the bedside drainage adapter 754 may be fit into the opening 756 by applying opposed, inwardly directed forces 'F' (shown as arrows in FIG. 39) to the outlet body 750. The bedside drainage adapter 754 may include a mounting loop 704 and a strap 705. In one embodiment, the mounting loop 704 may be made from an elastic material. However, other materials are also envisioned so long as the mounting loop 704 is sized to correspond to an outer circumference or perimeter of the outlet body 750 The bedside drainage adapter 754 may also include an inner extension 790 configured for positioning within the opening 756 and a lip 796 for abutting an axial end face of the outlet body 750. In one embodiment, the lip 796 may extend over an exterior surface along a portion of the length of the outlet body 750.

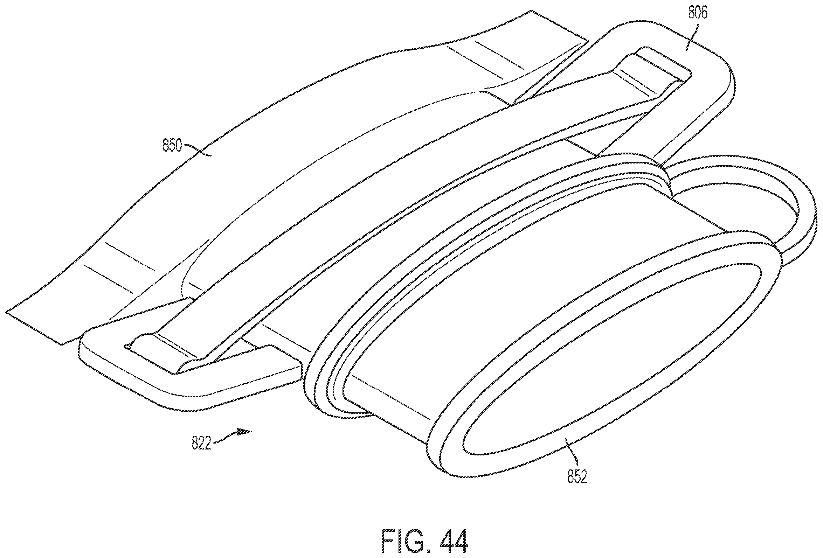

FIG. 44 shows an interface assembly 822 having an outlet body 850 and cap 852 according to another embodiment described herein. Referring to FIG. 44, the outlet body of, for example, the embodiments shown in FIGS. 29-43, may further include a closure mechanism 806 extending around the outlet body 850. The closure mechanism 806 may be, for example, a clamp, or the like. The closure mechanism 806 may exert a clamping or closing force on the outlet body 850 to further assist in sealing the outlet body 850 against unintentional egress of contents from the ostomy pouch 12. The cap 852 may be disposed at least partially within the outlet body 850 to close the outlet body 850.

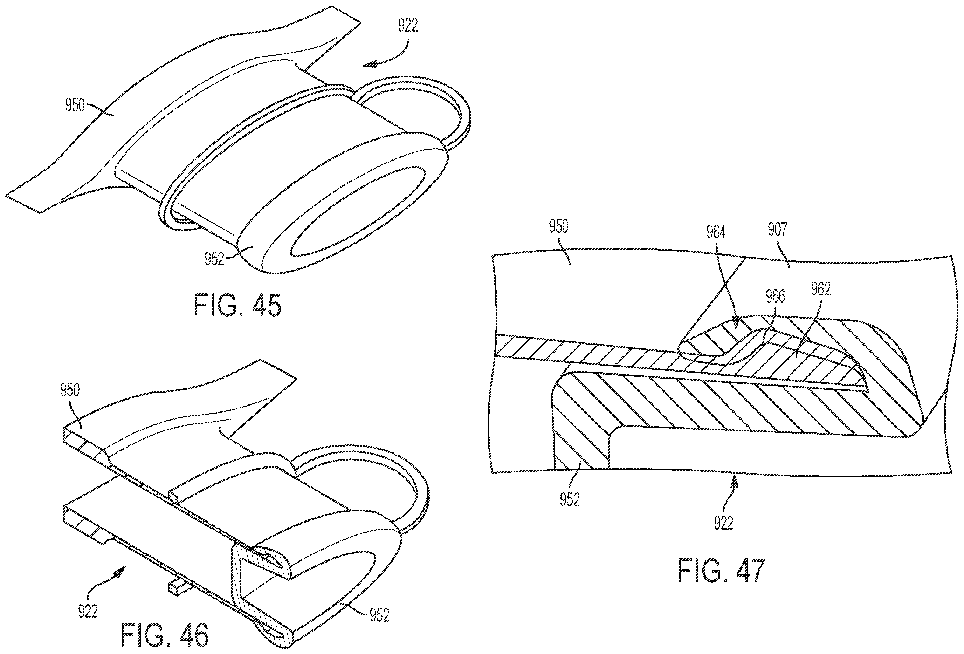

FIGS. 45-47 show an interface assembly 922 having an outlet body 950 and cap 952 according to another embodiment described herein. Referring to FIGS. 45-47, the cap 952, such as that described in the embodiments shown in FIGS. 29-43, may further include an inwardly projecting collar 907 configured to engage an exterior surface of the outlet body 950. The inwardly projecting collar 907 may have an inwardly projecting second shoulder 964 and the exterior surface of the outlet body 950 may have an outwardly projecting first shoulder 962. The inwardly projecting shoulder 964 of the cap 952 and the outwardly projecting first shoulder 962 of the outlet body 950 may interlock or engage as they slide past one another to form a perimeter seal 966.

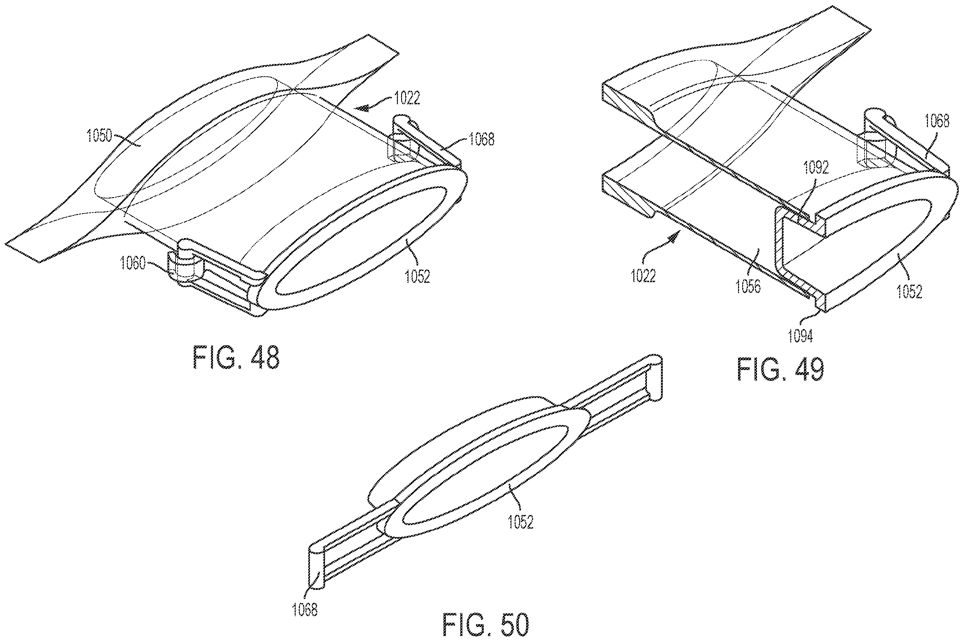

FIGS. 48-52 show an interface assembly 1022 having an outlet body 1050, cap 1052 and bedside drainage adapter 1054 according to another embodiment described herein. Referring to FIGS. 48-52, the outlet body 1050 may be formed with an opening 1056 at the discharge end and include a first latch 1060. In one embodiment, the first latch 1060 may include a pair of outwardly projecting hooks or catches. The cap 1052 is configured to be fitted in the opening 1056 to substantially seal the opening 1056. The cap 1052 may include an inner extension 1092 received in the opening 1056 and a lip 1094 configured to abut an axial end face of the outlet body 1050. The cap 1052 also includes a second latch 1068 configured to engage the first latch 1060. In one embodiment, the second latch 1068 includes a pair of loop or hook-like tabs movable on a living hinge to engage the first latch 1060. The cap 1052 may be removed and replaced with the bedside drainage adapter 1054 to allow for draining of the ostomy pouch 12. The bedside drainage adapter 1054 includes an inner extension 1090 for positioning in the opening 1056 and a lip 1096 for abutting an axial end face of the outlet body 1050. Further, the bedside drainage adapter 1054 may include a third latch 1078 for engaging the first latch 1060. In one embodiment, the third latch 1078 includes a pair of loop or hook-like movable on a living hinge to engage the first latch 1060.

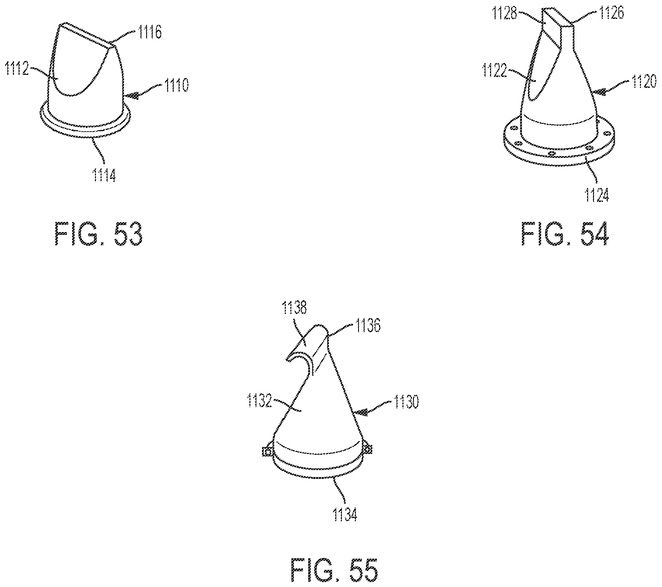

FIGS. 53-55 show different embodiments of a valve configured for positioning in the outlet body described in the embodiments above. In general, the valve may be secured in an internal passageway of the outlet body. Preferably, the valve is sealed to the inner wall or surface of the outlet body completely about a periphery or circumference of the inner wall. The valve is generally formed by a valve body having an open end and a valve end that is actuatable between an opened position and a closed position. In one embodiment, the valve end may generally be formed as a slit in the material of valve body, urged to a closed position where flow of fluid or other contents therethrough is restricted or prevented. A force can be applied generally to the valve end to open the slit, thereby permitting flow fluid or other contents therethrough. The valve may also include a mounting structure, such as a collar or the like, to secure the valve to the outlet body. In some embodiments, the valve may be formed integrally and continuously, as one piece, with the outlet body.

Referring to FIG. 53, in one embodiment, a valve 1110 includes a valve body 1112 having an open end 1114 and a valve end 1116 actuatable between open and closed positions. The valve end 1116 includes a normally closed slit configured to restrict or prevent flow of fluid or other contents therethrough. The slit may be moved to an open condition in response to application of a force, thereby permitting flow of fluid or contents therethrough. In one embodiment, the valve 1110 may generally be formed as a duckbill valve having angled sides coming together at the same angle as the bills. The slit may be moved to the open condition by, for example, opposing inward lateral forces. That is, the valve 1110 may be actuated by squeezing opposite lateral sides.

Referring to FIG. 54, in one embodiment, a valve 1120 includes a valve body 1122 having an open end 1124 and a valve end 1126 actuatable between open and closed positions. The valve end 1126 includes a normally closed slit configured to restrict or prevent flow of fluid or other contents therethrough. The slit may be moved to an open condition in response to application of a force, thereby permitting flow of fluid or contents therethrough. In one embodiment, the valve 1120 may generally be formed as a duckbill valve having angled bills coming together at an extended flat tip section 1128. The slit may be formed at an end of the extended flat tip section. The slit may be moved to the open condition by application of inwardly directed squeezing forces on opposite lateral sides, for example.

Referring to FIG. 55, in one embodiment, a valve 1130 includes a valve body 1132 having an open end 1134 and a valve end 1136 actuatable between open and closed positions. The valve end 1136 may be formed as a curled over tip 1138 urged to a curled condition to close the valve 1130. The curled tip may be moved to generally straightened or extended position to open the valve 1130, thereby permitting flow of fluid or contents therethrough.

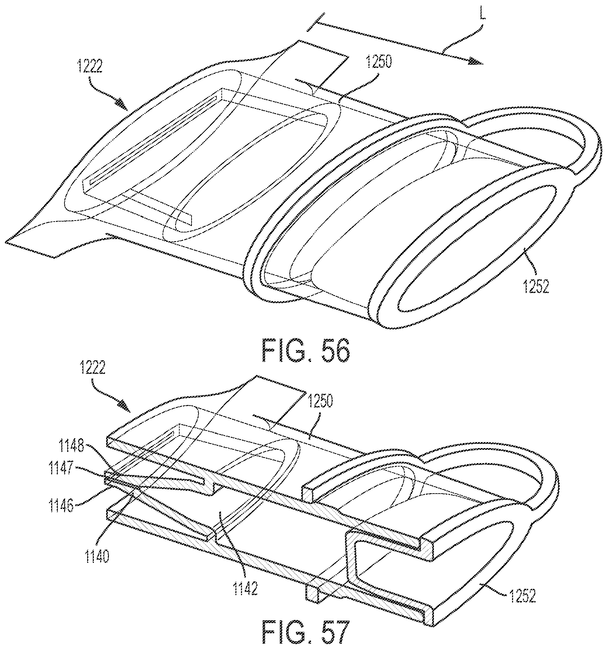

FIGS. 56 and 57 show an interface assembly 1222 having an outlet body 1250 and a cap 1252 according to another embodiment described herein. Referring to FIGS. 56 and 57, the outlet body 1250 include a valve 1140 disposed therein. The valve 1140 may be any of valves 1110, 1120, 1130 described in the embodiments above with respect to FIGS. 53-55, or other similar, suitable valves. The valve 1140 may generally be formed as a duckbill valve having a valve end 1146 disposed upstream from an open end 1142 thereof. It is understood that the terminology "upstream" generally refers to a direction of the flow of contents out of ostomy pouch 12 through outlet body 1250 during draining of the ostomy pouch 12. The valve end 1146 may be formed having a slit 1147 at a tip section 1148. The slit may be normally urged to a closed position to prevent or limit flow through the valve 1140, and can be moved to an opened position, allow flow through the valve, by applying opposite, inwardly directed forces at lateral side of the valve 1140, for example, through the outlet body 1250. The cap 1252 may be formed substantially the same as the caps 752, 852 disclosed in the embodiments shown in FIGS. 29-38, 40, 41, 43 and 44, described above. In one embodiment, the outlet body 1250 may extend about 53 mm in a length direction 'L' (see FIG. 56). However, it is understood that this length is included for the purposes of example, and does not limit this embodiment, or other embodiments described herein to such a length.

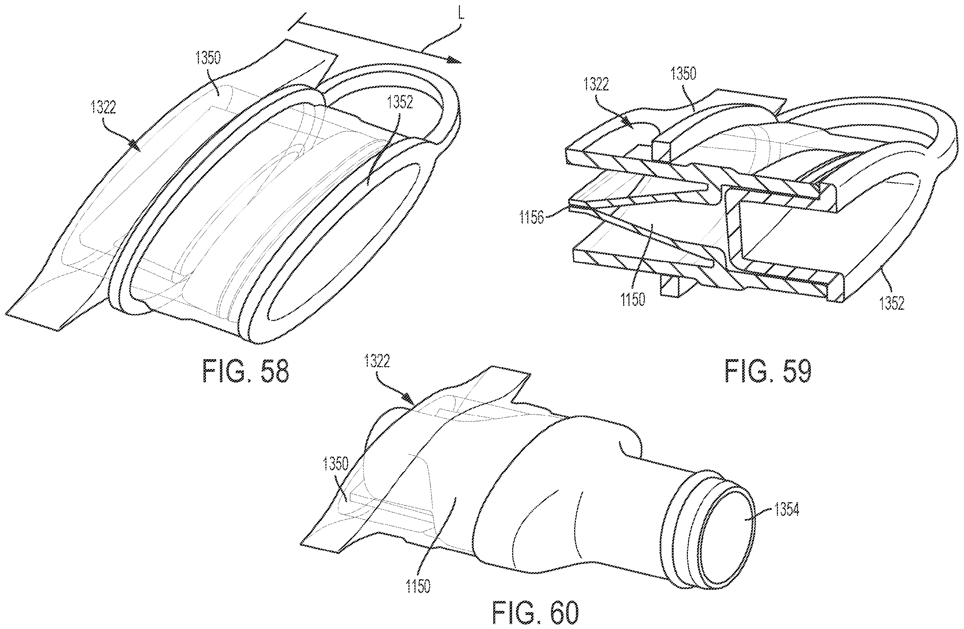

FIGS. 58-60 show examples of an interface assembly 1322 having an outlet body 1350, a cap 1352 and a bedside drainage adapter 1354 according to another embodiment described herein. As best shown in FIG. 59, the outlet body 1350 may have a valve 1150 disposed therein, similar to the valve described above and shown in FIG. 56 and 57. It understood that alternative valves are envisioned as well, including those shown in FIGS. 53-55 and described above. The cap 1352 may be formed substantially the same as the caps 752, 852 in the embodiments shown in FIGS. 29-38, 40, 41, 43, 44 and described above. In FIGS. 58-60, the outlet body 1350 may extend about 30 mm in a length direction 'L'(see FIG. 58), but is not limited thereto. Referring to FIG. 60, the cap 1352 may be replaced with the bedside drainage adapter 1352 to allow for drainage of the ostomy pouch 12. In one embodiment, the bedside drainage adapter 1352 may be fitted over the outlet body 1350 and a valve end 1156 of the valve 1150 may be opened to accommodate drainage of the ostomy pouch 12.

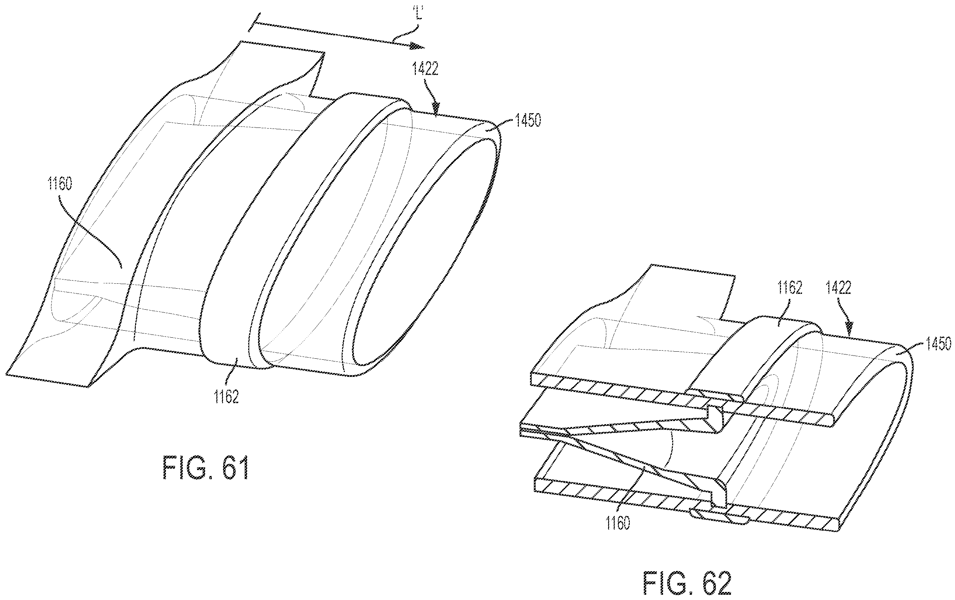

FIGS. 61 and 62 show examples of an interface assembly 1422 having an outlet body 1450 with a valve 1160 disposed therein. The valve 1160 may be similar or identical to valves described in the embodiments above and shown in FIGS. 53-60, or other suitable valves. However, referring to FIGS. 61 and 62, the valve 1160 may include a mounting structure 1162 positioned externally of the outlet body 1450. That is, the valve 1160, at one end, may extend through the outlet body 1450 to a mounting structure 1162 positioned at an external surface of the outlet body 1450. In one embodiment, the mounting structure 1162 may be collar extending about the outlet body 1450.

For example, with further references to FIGS. 61 and 62, the valve 1160 may be co-molded with the outlet body 1450. In one embodiment, the outlet body 1450 may be formed from thermoplastic elastomer (TPE) or other material with suitable elastic memory, while the valve 1160 is formed from silicone. The valve 1160 may be molded around the outlet body 1450. In one embodiment, the outlet body 1450 may extend about 30 mm in a length direction 'L' (see FIG. 61), but is not limited thereto.

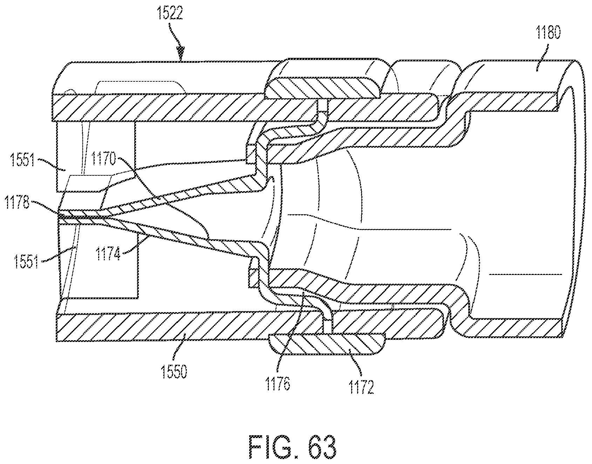

FIG. 63 shows an example of an interface assembly 1522 having an outlet body 1550 and a valve 1170 according to another embodiment described herein. Referring to FIG. 63 the outlet body 1550 and valve 1170 may be formed similarly to the outlet body 1450 and valve 1160, respectively, described above with respect to the embodiment shown in FIGS. 61 and 62. As such, the outlet body 1550 may be molded from TPE or other similar material having a suitable elastic memory. The valve 1170 may be co-molded in silicone around the outlet body 1550, such that the valve 1170 includes a mounting structure 1172 positioned on an exterior of the outlet body 1550, extending to an interior of the outlet body 1550 where a body 1174 of the valve 1170 is disposed. The valve body includes an open end 1176 and a valve end 1178 similar to the valves described in the embodiments above.

In addition, the outlet body 1550 may include internally disposed ribs 1551. The ribs 1551 may be substantially oppositely positioned from one another and are configured to apply pressure to the valve end 1178 of the valve 1170. For example, the ribs 1551 may apply pressure to opposed duckbills of the valve 1170 at or near a tip or extended tip portion of the valve 1170. Accordingly, the ribs 1551 may hold the valve end 1178 in a closed position to prevent or limit inadvertent opening of the valve 1170.

Referring to still to FIG. 63, the valve 1170 may be actuated from the closed condition to an open condition by way of a valve slider 1180. The valve slider 1180 may extend within the outlet body 1550 and connect to the valve 1170, for example, at or near the open end 1176. The valve slider 1180 may be formed from a TPE material, or a similar material having suitable elastic memory. In use, the valve end 1178 may be seated against the ribs 1551 within the outlet body 1550. To drain the ostomy pouch 12, a user pulls the valve slide 1180 outward (i.e., generally axially away from the axial end of the outlet body in which it is positioned) which causes side extension walls of the valve 1170 to deflect or roll, thereby moving the valve end 1178 generally in an axial direction away from the ribs 1551, such that the ribs 1551 no restrict opening of the valve 1170. The valve 1170 may then be actuated, i.e., moved to the open condition, for example, by applying oppositely directed inward forces at lateral sides of the valve end 1178. In one embodiment, the valve slider 1180 may serve as a bedside drainage adapter. In another embodiment, the bedside drainage adapter may be coupled to the valve slider 1180. In still another embodiment, the valve slider 1180 may be removed and a bedside drainage adapter may be coupled to the valve 1170 or outlet body 1550.

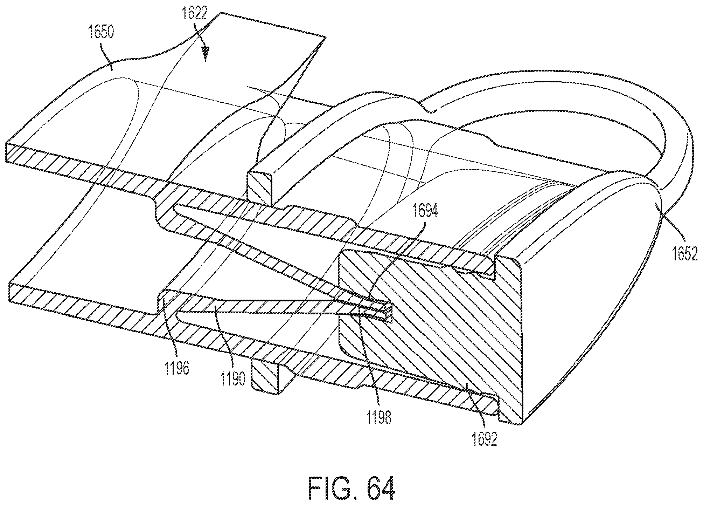

FIG. 64 shows an interface assembly 1622 having an outlet body 1650 and a cap 1652 according to another embodiment described herein. Referring to FIG. 64, the outlet body 1650 may include a valve 1190 substantially similar to the valves described in the embodiments above and shown in FIGS. 53-63. However, in this embodiment, the position of the valve 1190 may be reversed such that a valve end 1198 is generally downstream from an open end 1196 of the valve 1190. Here, "downstream" generally refers to a direction of flow of the fluid or contents as they are drained from the ostomy pouch 12 through the outlet body 1650. The cap 1652 may be formed similarly to the caps described in the embodiments above and shown, for example, in FIGS. 29-38, 40, 41, 43, and 44. However, in this embodiment, the cap 1652 may be formed having a generally solid closure portion 1692. The closure portion 1692 may include an open slot 1694 externally positioned on an inward facing surface thereof. The open slot 1694 is configured to receive the valve end 1198 of the valve 1190 to substantially hold the valve 1190 against opening.

To actuate the valve 1190, the cap 1652 may be removed from the outlet body 1650, thereby removing the slot 1694 from the valve end 1198. In this condition, the valve end 1198, formed as duck bills, for example, may elastically deflect to an open condition upon application of the predetermined force(s).

Accordingly, the embodiments above, an ostomy pouch may be configured for normal daily use or for night use. In normal daily use, the outlet of the ostomy pouch may be secured in a closed condition, for example by rolling or folding the outlet. Alternatively, or in addition, the ostomy pouch may be maintained in a closed condition by way of an outlet body connected to the outlet and a cap disposed over and/or partially within the outlet body to seal the outlet body against egress of the contents of the collection pouch. The outlet body may include a slit actuatable between opened and closed positions, thereby forming a squeeze valve. The slit may also be moved between opened and closed conditions, for example, by applying opposed inwardly directed forces to the outlet body.

To drain contents from the ostomy pouch, the outlet may be moved from the closed condition to the open condition by unrolling or unfolding. The cap may be removed from the outlet body bedside drainage adapter. Contents of the ostomy pouch may then be drained through the opening or slit of the outlet body, when the slit is moved to the open condition.

For bedside or night use, where increased storage capacity may be desirable, the ostomy pouch may be used as part of a drainable ostomy system. In a drainable system, such as a bedside or night drainage system, the outlet is moved to the open condition, as described above, so that the ostomy pouch may be drained. In addition, the cap may be replaced with the bedside drainage adapter. The bedside drainage adapter is coupled to and may be partially disposed within the slot or opening of the outlet body to maintain that outlet body in an open condition. The bedside drainage adapter connects the outlet body to a hose or tube, which, in turn, is connected to a bedside drainage bag to provide increased capacity. Accordingly, contents may be drained from the ostomy pouch, through the outlet, the outlet body, the bedside drainage adapter, the tube and into the bedside drainage bag.

Thus, the interface assembly is operable in a first state where the cap is engaged with the outlet body to substantially seal the outlet body against egress of the contents of the ostomy pouch therethrough. The interface assembly is also operable in a second state where the cap is removed from the outlet body and the bedside drainage adapter is engaged with the outlet body in manner that allows for flow of the contents of the ostomy pouch through the outlet body and the bedside drainage adapter. For example, in the second state, the bedside drainage adapter may also be coupled to a hose or tube which is coupled to the bedside drainage bag, such that the contents may flow from the ostomy pouch through the outlet body, the bedside drainage adapter, the tube and into the bedside drainage bag.

The bedside drainage bag may be translucent or transparent, or include translucent or transparent sections so that wearer or caretaker may visually inspect a volume of contents within the bedside drainage bag. In addition, the bedside drainage bag may include volume markers so that a specific volume of contents may be observed and measured. Further still, the bedside drainage system may be easily cleaned and/or components replaced by way of a quick release bag connector connecting the hose to the bedside drainage bag and the bedside drainage adapter connecting the hose to the ostomy pouch. The bedside drainage system also includes a hanger disposed, for example, on the bedside drainage bag, so that the bag may be supported on an adjacent structure, such as a bed, rack or the like. The hose or tube may also have an increased diameter compared to conventional ostomy drainage hoses or tubes, so as to limit or prevent blockages caused by contents within with tube.

The components of the drainable ostomy system described above may be formed from different materials or combinations of different materials, including, but not limited to, TPE, ethylene-vinyl acetate (EVA), polypropylene (PP), polyamide (PA), vinylidene chloride, including polyvinylidene chloride (PVDC), sold as SARAN. For example, in one embodiment, the bag connector may be made from TPE, the tube may be made from EVA, the bedside drainage adapter may be made from EVA and/or TPE, the ostomy pouch and the bedside drainage bag may be made from films, either clear, opaque, or combinations thereof, and may be formed of SARAN or similar materials. In addition, the hanger may be made from PP or PA, and the outlet body may be made from TPE. However, it is understood the present disclosure is not limited to these materials, and other suitable materials are also envisioned.

It is understood that the features described with respect to any of the embodiments above or shown in the drawings may be implemented, used together with, or replace features described in any of the other embodiments above, as suitable. It is also understood that description of some features may be omitted in some embodiments, where similar or identical features are discussed in other embodiments.

Further, it is understood that although the outlet body, cap, bedside drainage adapter and valve are described above in conjunction with an ostomy pouch for use in a bedside or night drainage system, that these features, i.e., the outlet body, cap, bedside drainage adapter and/or valve, may be used with an ostomy pouch independent from the bedside drainage system described herein. That is, the outlet body, cap, bedside drainage adapter and/or valve are not limited to use only with the bedside drainage system described herein. In addition, in the various embodiments described above and shown in the drawings, the cap is configured to couple with the outlet body so as to substantially or completely close and/or seal the outlet body against inadvertent leakage. In some embodiments, the cap may fit over the outlet body, while in other embodiments, the cap may fit within the outlet body, similar to a plug. In other embodiments, the cap may extend both within the outlet body and outside of the outlet body. For example, according to one embodiment, the cap may form a seal around both an inner periphery of the outlet body and an outer periphery.

In the present disclosure, the words "a" or "an" are to be taken to include both the singular and the plural. Conversely, any reference to plural items shall, where appropriate, include the singular.

## Claims

1. A drainable ostomy system (10) comprising:
an ostomy pouch (12) having an internal collection area (24) and an outlet (26) for draining contents from the collection area (24);
an interface assembly (22, 122, 622) comprising:
an outlet body operably connected to the outlet (26), the outlet body (150, 650, 750) having a discharge end (158), wherein the outlet body (150) is formed with a slit (156), wherein the slit (156) is configured to be selectively movable between an open condition and a closed condition, to selectively allow or prevent drainage of the contents from the ostomy pouch (12) through the slit (156);
a cap (152) configured for removable coupling with the outlet body (150, 650, 750), the cap (152) configured to close the discharge end (156) of the outlet body (150, 650, 750); and
a bedside drainage adapter (154, 654) configured for removable coupling with the outlet body (150, 650, 750),
wherein the cap (152) and the bedside drainage adapter (154, 654) are interchangeably coupled to the outlet body (150, 650, 750), **characterised in that**
the slit is formed at the discharge end (158) of the outlet body (150) and the discharge end (158) is urged to and held in a closed condition when the cap is coupled to the outlet body.

2. The drainable ostomy system (10) of claim 1, wherein the bedside drainage adapter (154) is releasably coupled with the outlet body (150, 650, 750), and the system further comprises:
a tube (16) coupled to the bedside drainage adapter (154, 654);
a bedside drainage bag (14) coupled to the tube (16) and configured to receive the contents of the ostomy pouch (12) via the outlet body (150, 650, 750), the bedside drainage adapter (154) and the tube (16).

3. The drainable ostomy system (10) of claim 2, wherein the bedside drainage bag (14) includes one or more translucent or transparent sections allowing for visual inspection of an interior storage area of the bedside drainage bag (14).

4. The drainable ostomy system of claim 3, wherein the bedside drainage bag (14) further includes a hanger (18) configured to attach the bedside drainage bag (14) to an adjacent structure.

5. The drainable ostomy system (10) of claim 3, wherein the bedside drainage bag (14) further comprises volume markings (44) at the one or more translucent or transparent sections allowing for visual measurement of a volume of contents within the bag (14).

6. The drainable ostomy system (10) of the claim 1, wherein the outlet body (150, 650, 750) further includes a first latch (160), the cap (152) further includes a second latch (168) and the bedside drainage adapter (154, 654) includes a third latch (178), wherein the first latch (160) is configured to engage, interchangeably, with the second latch (168) and the third latch (178).

7. The drainable ostomy system (10) of claim 1, wherein the outlet (26) is foldable or rollable between an open condition to allow for drainage of the contents from the ostomy pouch (12) and a closed condition to substantially prevent drainage of the contents from the ostomy pouch (12).

8. The drainable ostomy system (10) of claim 1, wherein the outlet body (150, 650) includes a first shoulder (162), the cap (152) includes a second shoulder (164) and the bedside drainage adapter (154, 654) includes a third shoulder (174), wherein the first shoulder (162) is configured to engage, interchangeably, with the second shoulder (164) and the third shoulder (174) to form a perimeter seal (166).

9. The drainable ostomy system (10) of claim 1, wherein the cap (152) further includes a mounting loop (702) extending about an outer periphery of the outlet body (150, 650, 750) to secure the cap (152) to the outlet body (150, 650, 750).

10. The drainable ostomy system (10) of claim 1, wherein the bedside drainage adapter (154, 654, 754) further includes a mounting loop (702) extending about an outer periphery of the outlet body (150, 650, 750) to secure the bedside drainage adapter to the outlet body.

11. The drainable ostomy system (10) of claim 1, wherein the outlet body comprises a base (480, 580) having an end part (482, 583) operably connected thereto.

12. The drainable ostomy system (10) of claim 1, further comprising a valve (1110, 1120) disposed at least partially within the outlet body, wherein the slit is formed at an end portion of the valve.

## Patentansprüche

1. Entleerbares Ostomiesystem (10), umfassend:
einen Ostomiebeutel (12), der einen inneren Sammelbereich (24) und einen Auslass (26) zum Entleeren von Inhalt aus dem Sammelbereich (24) aufweist;
eine Schnittstellenanordnung (22, 122, 622), umfassend:
einen Auslasskörper, der mit dem Auslass (26) wirkverbunden ist, wobei der Auslasskörper (150, 650, 750) ein Auslassende (158) aufweist, wobei der Auslasskörper (150) mit einem Schlitz (156) ausgebildet ist, wobei der Schlitz (156) dazu konfiguriert ist, selektiv zwischen einem geöffneten Zustand und einem geschlossenen Zustand bewegbar zu sein, um ein Entleeren des Inhalts aus dem Ostomiebeutel (12) durch den Schlitz (156) selektiv zu ermöglichen oder zu verhindern;
eine Kappe (152), die zum lösbaren Koppeln mit dem Auslasskörper (150, 650, 750) konfiguriert ist, wobei die Kappe (152) zum Schließen des Auslassendes (156) des Auslasskörpers (150, 650, 750) konfiguriert ist; und
einen am Bett angebrachten Entleerungsadapter (154, 654), der zum lösbaren Koppeln mit dem Auslasskörper (150, 650, 750) konfiguriert ist,
wobei die Kappe (152) und der am Bett angebrachte Entleerungsadapter (154, 654) austauschbar mit dem Auslasskörper (150, 650, 750) gekoppelt sind, **dadurch gekennzeichnet, dass**
der Schlitz am Auslassende (158) des Auslasskörpers (150) ausgebildet ist und das Auslassende (158) in einen geschlossenen Zustand gedrängt und in diesem gehalten wird, wenn die Kappe mit dem Auslasskörper gekoppelt ist.

2. Entleerbares Ostomiesystem (10) nach Anspruch 1, wobei der am Bett angebrachte Entleerungsadapter (154) lösbar mit dem Auslasskörper (150, 650, 750) gekoppelt ist und das System ferner umfasst:
einen Schlauch (16), der mit dem am Bett angebrachten Entleerungsadapter (154, 654) gekoppelt ist;
einen mit dem Schlauch (16) gekoppelten, am Bett angebrachten Entleerungsbeutel (14), der zum Aufnehmen des Inhalts des Ostomiebeutels (12) über den Auslasskörper (150, 650, 750), den am Bett angebrachten Entleerungsadapter (154) und den Schlauch (16) konfiguriert ist.

3. Entleerbares Ostomiesystem (10) nach Anspruch 2, wobei der am Bett angebrachte Entleerungsbeutel (14) einen oder mehrere transluzente oder transparente Abschnitte beinhaltet, die eine visuelle Inspektion eines inneren Aufbewahrungsbereichs des am Bett angebrachten Entleerungsbeutels (14) ermöglichen.

4. Entleerbares Ostomiesystem nach Anspruch 3, wobei der am Bett angebrachte Entleerungsbeutel (14) ferner eine Hängevorrichtung (18) beinhaltet, die zum Anbringen des am Bett angebrachten Entleerungsbeutels (14) an einer benachbarten Struktur konfiguriert ist.

5. Entleerbares Ostomiesystem (10) nach Anspruch 3, wobei der am Bett angebrachte Entleerungsbeutel (14) ferner Volumenmarkierungen (44) an dem einen oder den mehreren transluzenten oder transparenten Abschnitten umfasst, die eine visuelle Messung eines Volumens des Inhalts innerhalb des Beutels (14) ermöglichen.

6. Entleerbares Ostomiesystem (10) nach Anspruch 1, wobei der Auslasskörper (150, 650, 750) ferner einen ersten Riegel (160) beinhaltet, die Kappe (152) ferner einen zweiten Riegel (168) beinhaltet und der am Bett angebrachte Entleerungsadapter (154, 654) einen dritten Riegel (178) beinhaltet, wobei der erste Riegel (160) dazu konfiguriert ist, mit dem zweiten Riegel (168) und dem dritten Riegel (178) austauschbar in Eingriff zu stehen.

7. Entleerbares Ostomiesystem (10) nach Anspruch 1, wobei der Auslass (26) zwischen einem geöffneten Zustand, um ein Entleeren des Inhalts aus dem Ostomiebeutel (12) zu ermöglichen, und einem geschlossenen Zustand, um ein Entleeren des Inhalts aus dem Ostomiebeutel (12) im Wesentlichen zu verhindern, faltbar oder rollbar ist.

8. Entleerbares Ostomiesystem (10) nach Anspruch 1, wobei der Auslasskörper (150, 650) eine erste Schulter (162) beinhaltet, die Kappe (152) eine zweite Schulter (164) beinhaltet und der am Bett angebrachte Entleerungsadapter (154, 654) eine dritte Schulter (174) beinhaltet, wobei die erste Schulter (162) dazu konfiguriert ist, austauschbar mit der zweiten Schulter (164) und der dritten Schulter (174) in Eingriff zu kommen, um eine Umfangsdichtung (166) zu bilden.

9. Entleerbares Ostomiesystem (10) nach Anspruch 1, wobei die Kappe (152) ferner eine Befestigungsschlaufe (702) beinhaltet, die sich um einen Außenumfang des Auslasskörpers (150, 650, 750) erstreckt, um die Kappe (152) an dem Auslasskörper (150, 650, 750) zu befestigen.

10. Entleerbares Ostomiesystem (10) nach Anspruch 1, wobei der am Bett angebrachte Entleerungsadapter (154, 654, 754) ferner eine Befestigungsschlaufe (702) beinhaltet, die sich um einen Außenumfang des Auslasskörpers (150, 650, 750) erstreckt, um den am Bett angebrachten Entleerungsadapter am Auslasskörper zu befestigen.

11. Entleerbares Ostomiesystem (10) nach Anspruch 1, wobei der Auslasskörper einen Boden (480, 580) umfasst, der ein Endteil (482, 583) aufweist, das mit diesem in Wirkverbindung steht.

12. Entleerbares Ostomiesystem (10) nach Anspruch 1, ferner umfassend ein Ventil (1110, 1120), das mindestens teilweise innerhalb des Auslasskörpers angeordnet ist, wobei der Schlitz an einem Endabschnitt des Ventils ausgebildet ist.

## Revendications

1. Système de stomie vidangeable (10) comprenant :
une poche de stomie (12) ayant une zone de collecte interne (24) et une sortie (26) permettant de vidanger le contenu de la zone de collecte (24) ;
un ensemble interface (22, 122, 622) comprenant :
un corps de sortie relié fonctionnellement à la sortie (26), le corps de sortie (150, 650, 750) ayant une extrémité d'évacuation (158), dans lequel le corps de sortie (150) est formé avec une fente (156), dans lequel la fente (156) est conçue pour être sélectivement mobile entre un état ouvert et un état fermé, afin de permettre ou d'empêcher sélectivement la vidange du contenu de la poche de stomie (12) à travers la fente (156) ;
un capuchon (152) conçu pour un couplage amovible avec le corps de sortie (150, 650, 750), le capuchon (152) étant conçu pour fermer l'extrémité d'évacuation (156) du corps de sortie (150, 650, 750) ; et
un adaptateur de vidange côté lit (154, 654) conçu pour un couplage amovible avec le corps de sortie (150, 650, 750),
dans lequel le capuchon (152) et l'adaptateur de vidange côté lit (154, 654) sont couplés de manière interchangeable au corps de sortie (150, 650, 750), **caractérisé en ce que**
la fente est formée au niveau de l'extrémité d'évacuation (158) du corps de sortie (150) et l'extrémité d'évacuation (158) est poussée vers et maintenue dans un état fermé lorsque le capuchon est couplé au corps de sortie.

2. Système de stomie vidangeable (10) selon la revendication 1, dans lequel l'adaptateur de vidange côté lit (154) est couplé de manière libérable au corps de sortie (150, 650, 750), et le système comprend également :
un tube (16) couplé à l'adaptateur de vidange côté lit (154, 654) ;
un sac de vidange côté lit (14) couplé au tube (16) et conçu pour recevoir le contenu de la poche de stomie (12) par l'intermédiaire du corps de sortie (150, 650, 750), de l'adaptateur de vidange côté lit (154) et du tube (16).

3. Système de stomie vidangeable (10) selon la revendication 2, dans lequel le sac de vidange côté lit (14) comporte une ou plusieurs sections translucides ou transparentes permettant l'inspection visuelle d'une zone de stockage intérieure du sac de vidange côté lit (14).

4. Système de stomie vidangeable selon la revendication 3, dans lequel le sac de vidange côté lit (14) comporte également un dispositif de suspension (18) conçu pour fixer le sac de vidange côté lit (14) à une structure adjacente.

5. Système de stomie vidangeable (10) selon la revendication 3, dans lequel le sac de vidange côté lit (14) comprend également des marques de volume (44) au niveau de la ou des sections translucides ou transparentes permettant la mesure visuelle d'un volume de contenu à l'intérieur du sac (14).

6. Système de stomie vidangeable (10) selon la revendication 1, dans lequel le corps de sortie (150, 650, 750) comporte également un premier verrou (160), le capuchon (152) comporte également un deuxième verrou (168) et l'adaptateur de vidange côté lit (154, 654) comporte un troisième verrou (178), dans lequel le premier verrou (160) est conçu pour venir en prise, de manière interchangeable, avec le deuxième verrou (168) et le troisième verrou (178).

7. Système de stomie vidangeable (10) selon la revendication 1, dans lequel la sortie (26) est pliable ou roulable entre un état ouvert pour permettre la vidange du contenu de la poche de stomie (12) et un état fermé pour empêcher sensiblement la vidange du contenu de la poche de stomie (12).

8. Système de stomie vidangeable (10) selon la revendication 1, dans lequel le corps de sortie (150, 650) comporte un premier épaulement (162), le capuchon (152) comporte un deuxième épaulement (164) et l'adaptateur de vidange côté lit (154, 654) comporte un troisième épaulement (174), dans lequel le premier épaulement (162) est conçu pour venir en prise, de manière interchangeable, avec le deuxième épaulement (164) et le troisième épaulement (174) afin de former un joint périmétrique (166) .

9. Système de stomie vidangeable (10) selon la revendication 1, dans lequel le capuchon (152) comporte également une boucle de montage (702) s'étendant autour d'une périphérie externe du corps de sortie (150, 650, 750) pour fixer le capuchon (152) au corps de sortie (150, 650, 750).

10. Système de stomie vidangeable (10) selon la revendication 1, dans lequel l'adaptateur de vidange côté lit (154, 654, 754) comporte également une boucle de montage (702) s'étendant autour d'une périphérie externe du corps de sortie (150, 650, 750) pour fixer l'adaptateur de vidange côté lit au corps de sortie.

11. Système de stomie vidangeable (10) selon la revendication 1, dans lequel le corps de sortie comprend une base (480, 580) à laquelle est reliée fonctionnellement une partie d'extrémité (482, 583).

12. Système de stomie vidangeable (10) selon la revendication 1, comprenant également un robinet (1110, 1120) disposé au moins partiellement à l'intérieur du corps de sortie, dans lequel la fente est formée au niveau d'une partie d'extrémité du robinet.
